Europäisches Patentamt

(19) European Patent Office

Office européen des brevets

(11) Numéro de publication: **0 000 968**
**B1**

(12) **FASCICULE DE BREVET EUROPEEN**

(45) Date de publication du fascicule de brevet: **14.10.81**

(21) Numéro de dépôt: **78200130.9**

(22) Date de dépôt: **04.08.78**

(51) Int. Cl.³: **A 61 K 33/24** //A61K31/19,
(A61K33/24, 33/32, 33/34)

(54) Composition pharmaceutique exerçant une action antiinfectieuse, antiinflammatoire et antiasthénique.

(30) Priorité: **11.08.77 FR 7724685**

(43) Date de publication de la demande:
**07.03.79 Bulletin 79/5**

(45) Mention de la délivrance du brevet:
**14.10.81 Bulletin 81/41**

(84) Etats Contractants Désignés:
**BE CH DE GB LU NL SE**

(56) Documents cités:
**BE - A - 674 532**

**VIDAL 1967, page 943;**
**"Cuivre-or-argent oligosol"**
**"Manganèse-cuivre-oligosol"**

(73) Titulaire: **Suck, Catherine Geneviève**
**10, rue Emile Morel**
**F-92330 Sceaux (FR)**
(73) Titulaire: **Lepeix, Liliane**
**7, rue Théophile Roussel**
**F-75012 Paris (FR)**

(72) Inventeur: **Suck, Catherine Geneviève**
**10, rue Emile Morel**
**F-92330 Sceaux (FR)**
Inventeur: **Lepeix, Liliane**
**7, rue Théophile Roussel**
**F-75012 Paris (FR)**

(74) Mandataire: **Nony, Michel et al,**
**Conseil en Brevets d'Invention 29 rue Cambacérès**
**F-75008 Paris (FR)**

Courier Press, Leamington Spa, England.

**0 000 968**

## Composition pharmaceutique exerçant une action antiinfectieuse, antiinflammatoire et antiasthénique

La présente invention a trait à de nouvelles compositions pharmaceutiques exerçant une action antiinfectieuse, antiinflammatoire et antiasthénique.

Il est connu dans l'art antérieur de recourir aux oligoéléments qui sont généralement des métaux que l'on trouve à l'état naturel aussi bien dans le règne minéral que dans le végétal ou le règne animal.

Leur rôle a été mis en évidence en particulier grâce aux recherches de Bertrand, Raulin et Javillier, (Institut Pasteur).

Il apparait en effet qu'ils interviennent dans de très nombreuses réactions biologiques notamment à titre de catalyseurs, ce qui les rend indispensables à l'équilibre biologique et donc à la vie. Cette intervention est donc essentiellement qualitative plus que quantitative dès lors que dans la plupart des cas leur présence n'est indispensable qu'en des quantités relativement faibles; leur réintroduction dans un organisme qui en est privé entraîne donc des activations ou rétablissements de fonctions provisoirement ralenties ou bloquées du fait d'une affection commençante, ou déclarée.

Les oligoéléments interviennent donc essentiellement, en les réactivant, sur les défenses organiques propres du patient de sorte qu'ils procurent à l'organisme la possibilité de recouvrer de lui-même son propre équilibre.

Il est ainsi décrit dans l'art antérieur et notamment dans le brevet Belge n°674.532 des compositions à base d'or et de composés de cuivre et d'argent destinés au traitement des états infectieux.

L'argent présente des propriétés bactéricides, le cuivre des propriétés antiinfectieuses et l'or des propriétés antiinflammatoires.

Il est également décrit dans l'art antérieur et notamment dans le document VIDAL 1967 page 943 des compositions à base de gluconate de manganèse et de gluconate de cuivre destinés au traitement des états infectieux chroniques où récidivants.

De telles compositions, bien que particulièrement efficaces dans les cas aigus, ne sont pas adaptées à d'autres types d'affections caractérisées par exemple par un état de fatigue général ou par leur chronicité. Il est nécessaire dans ces cas de recourir à un agent favorisant les oxydoréductions.

Les compositions conformes à la présente invention ont donc pour but d'agir sur le manque d'autodéfense, la fatigabilité physique et la lassitude psychique qui précèdent ou accompagnent les états chroniques, les affections récidivantes ou répétées, les convalescences ou la sénescence.

Dans les compositions conformes à la présente invention, l'or joue un rôle dans les réactions antitoxiques, antiinflammatoires et antirhumatismales. Le cuivre favorise l'élaboration d'anticorps et relance l'activité de l'acide ascorbique ce qui agit en particulier sur le fonctionnement des surrénales. De son côté, le manganèse, jouant un rôle d'oxydoréduction, intervient au niveau des métabolismes intermédiaires et en particulier dans la synthèse de l'hémoglobine, dans l'établissement de l'immunité et produit donc un effet antiasthénique.

L'or à l'etat colloïdal se révèle particulièrement compatible avec les sels de manganèse et de cuivre de sorte que cela constitue un avantage certain pour leur association.

Plus particulièrement, on a obtenu les résultats les plus satisfaisants avec l'or colloïdal et des sels de manganèse et de cuivre, notamment le gluconate de manganèse

$$Mn = (OOC — HCOH — HOCH — HCOH — HCOH — CH2OH)2$$
et le gluconate de cuivre
$$Cu = (OOC — HCOC — HOCH — HCOH — HCOH — CH2OH)2$$
dans les teneurs suivantes pour 100 ml
Or colloïdal de 0,0003 à 0,0012 g
Gluconate de Mn de 0,015 à 0,060 g
Gluconate de Cu de 0,015 à 0,060 g.

Qui plus est, l'association avec la gomme arabique assure la stabilisation de l'or colloïdal.

Pour mieux faire comprendre les caractéristiques techniques et les avantages de la présente invention, on va en décrire un exemple de réalisation étant bien entendu qui celui-ci n'est pas limitatif quant à son mode de mise en oeuvre et aux applications qu'on peut en faire.

On a préparé la composition suivante:

| Eau distillée en Q.S.P. | Composition Centésimale/100 ml | Composition Unitaire/5 ml |
|---|---|---|
| Gluconate de manganèse | 0,030 g | 0,0015 g |
| Gluconate de cuivre | 0,030 g | 0,0015 g |
| Or colloïdal | 0,0006 g | 0,00003 g |
| Gomme arabique | 0,0001 g | 0,000005 g |

La composition est prescrite à raison de 1/2 à 2 prises unitaires par jour. Aucune toxicité n'a été relevée au cours des derniers essais.

On a administré à un homme de 45 ans souffrant d'arthrose 1 prise par jour pendant 12 jours; le traitement s'est révélé particulièrement efficace et on a pu noter un rétablissement de l'équilibre général avec disparition de la fatique.

On a également administré à une femme de 60 ans souffrant de rhumatismes chroniques 1/2 prise unitaire par jour pendant une série de 3 cures de 30 jours séparées par des intervalles de 30 jours. On a constaté les mêmes améliorations que dans le cas précédent.

Dans l'ensemble, on a constaté que cette composition se révélait d'autant plus active qu'elle était administrée dès les premiers signes de lassitude ou de fatique.

Si lors d'une première cure, la capacité réactionnelle propre est suffisamment rétablie pour que le patient retrouve son équilibre, il n'est généralement pas nécessaire de poursuivre.

Si par contre il s'agit de traiter un état ancien ou chronique ou une tendance à la récidive saisonnière, plusieurs cures sont parfois nécessaires effectuées de préférence aux périodes critiques qui se situent généralement en automne ou en hiver.

**Revendications**

1. Composition pharmaceutique caractérisée par le fait qu'elle contient pour 100 ml de solution aqueuse les teneurs suivantes:
   Or colloïdal de 0,0003 à 0,0012 g
   Gluconate de Manganèse de 0,015 à 0,060 g
   Gluconate de cuivre de 0,015 à 0,060 g.

2. Composition selon la revendication 1 caractérisée par le fait qu'elle contient de la gomme arabique.

3. Composition selon l'une quelconque des revendications 1 et 2 caractérisée par le fait qu'elle contient les teneurs suivantes:
   Or colloïdal 0,0006 g/100 ml
   Gluconate de manganèse 0,030 g/100 ml
   Gluconate de cuivre 0,030 g/100 ml
   Gomme arabique 0,0001 g/100 ml
   Eau distillée en quantité suffisante pour 100 ml.

**Claims**

1. A pharmaceutical composition containing in 100 ml of an aqueous solution the following amounts:
   Colloidal gold from 0,0003 to 0,0012 g
   Manganese gluconate from 0,015 to 0,060 g
   Copper gluconate from 0,015 to 0,060 g.

2. The composition of claim 1 which also contains arabic gum.

3. The composition of claim 1 or 2 which contains the following amounts:
   Colloidal gold 0,0006 g/100 ml
   Manganese gluconate 0,030 g/100 ml
   Copper gluconate 0,03 g/100 ml
   Arabic gum 0,0001 g/100 ml
   Distillated water sufficient to 100 ml.

**Patentansprüche**

1. Pharmazeutisches Mittel dadurch gekennzeichnet dass es in einer 100 ml wässerigen Lösung die folgende Mengen
   Kolloïdal Gold von 0,0003 bis 0,0012 g

**0 000 968**

Mangan Glukonat von 0,015 bis 0,060 g
Kupfer Glukonat von 0,015 bis 0,060 g
enthält.

2. Mittel gemäss Anspruch 1 dadurch gekennzeichnet dass es Arabin enthält.

3. Mittel gemäss Anspruch 1 oder 2 dadurch gekennzeichnet dass es die folgende Mengen
Kolloidal Gold 0,0006 g/100 ml
Mangan Glukonat 0,030 g/100 ml
Kupfer Glukonat 0,030 g/100 ml
Arabin 0,0001 g/100 ml
Destilliertes Wasser q.s.p. 100 ml enthält.